(19) Europäisches Patentamt
European Patent Office
Office européen des brevets

(11) EP 1 872 787 A1

(12) **EUROPEAN PATENT APPLICATION**

(43) Date of publication:
02.01.2008 Bulletin 2008/01

(51) Int Cl.:
*A61K 31/711* [(2006.01)]    *A61P 13/12* [(2006.01)]
*A61P 37/00* [(2006.01)]    *A61P 3/08* [(2006.01)]

(21) Application number: 06425436.0

(22) Date of filing: 27.06.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(71) Applicant: Gentium S.p.A.
22090 Villaguardia (Como) (IT)

(72) Inventors:
• Echart, Cinara
20040 Usmate-Velate (MI) (IT)

• Ferro, Luisa Iris
20121 Milano (IT)
• Iacobelli, Massimo
20152 Milano (IT)

(74) Representative: Pistolesi, Roberto et al
Dragotti & Associati srl
Via Turati 32
20121 Milano (IT)

(54) **Use of defibrotide for the inhibition of heparanase**

(57) A study has been carried out to verify the effect of Defibrotide on the activity and expression of Heparanase enzyme on myeloma tumor cells (U266) and human microvascular endothelial cells (HMEC).

The study has demonstrated that defibrotide can be effectively used for the manufacture of a medicament for the treatment of diseaseses which are positively affected by the inhibition of Heparanse and/or by the downregulation of Heparanse gene expression, such as autoimmune and/or inflammatory diseases.

**Figure 3**: Heparanase gene expression on HMEC cell stimulated with Defibrotide

EP 1 872 787 A1

**Description**

The scope of this study was to verify the effect of Defibrotide on the activity and expression of Heparanase enzyme on myeloma tumor cells (U266) and human microvascular endothelial cells (HMEC).

## 1. State of the art

The term defibrotide (hereinafter DF) normally identifies a polydeoxyribonucleotide that is obtained by extraction from animal and/or vegetable tissues (1, 2); the polydesoxyribo-nucleotide is normally used in the form of an alkali-metal salt, generally a sodium salt; it's CAS Registry Number is 83712-60-1.

DF is used mainly on account of its antithrombotic activity (3), although it can be used in other applications such as, for example, the treatment of acute renal insufficiency (4) and the treatment of acute myocardial ischaemia (5). DF is also used in the treatment of emergency clinical conditions, for example, for suppressing the toxicity correlated with high doses of chemotherapy regimens, in particular, the hepatic veno-occlusive syndrome (11,12); DF has been shown to have protective action towards apoptosis induced by fludarabine and towards the alloactivation of endothelial and epithelial cells, without also altering the antileukaemic effects of fludarabine (13); pre-clinical data also exists on the protective effects of DF that have been achieved in a model of endothelial damage mediated by lipopolysaccharide (14). DF has also recently revealed to be particularly effective as anti-tumor agent (10). Patents have been granted on the use of DF for treating HIV infections (9) and other diseases (8).

A method of manufacturing DF with uniform and well-defined physical/chemical characteristics and which is also free of possible undesirable side effects is described in United States patents (6, 7). In particular, DF manufactured according to these patents, which are both incorporated herein as a reference, is a polydeoxyribonucleotide corresponding to the following formula of random sequence:

$$P_{1-5},(dAp)_{12-24},(dGp)_{10-20},(dTp)_{13-26},(dCp)_{10-20}$$

wherein P=phosphoric radical, dAp=deoxyadenylic monomer, dGp=deoxyguanylic monomer, dTp=deoxythymidylic monomer, dcp=deoxycytidylic monomer;
and which, according to a preferred embodiment, presents the following chemico-physical properties:

- electrophoresis=homogeneous anodic mobility;
- extinction coefficient, $E_{1cm}^{1\%}$ at 260$\pm$1 nm =220$\pm$10°;
- extinction reaction, $E_{230}/E_{260}$ =0.45$\pm$0.04;
- coefficient of molar extinction (referred to phosphorus), (P)=7.750$\pm$500;
- rotary power reversible hyperchromicity, indicated as % in native DNA; h=$\pm$155;
- a purine: pyrimidine ratio of 0.95$\pm$0.5.

This polydeoxyribonucleotide, independently on whether it is obtained by extraction or synthetically, is the compound which is preferably used or the purposes of the present invention.

## 2. Background

Heparanase is an endoglycosidase, which degrades heparan sulphate side chains of heparan sulphate proteoglycans in the extracellular matrix (ECM). Heparanase plays an important role in ECM degradation, facilitating the migration and extravasation of tumor cells and inflammatory leukocytes. Upon degradation, Heparanase releases growth factors and cytokines that stimulate cell proliferation and chemotaxis (15,16).

Heparanase is highly expressed in myeloid leukocytes (i.e. neutrophils) in platelets and in human placenta. Human Heparanase was found to be upregulated in various types of primary tumors, correlating in some cases with increased tumor invasiveness and vascularity and with poor prospective survival (17). These observations, the anti-cancerous effect of Heparanase gene silencing and of Heparanase-inhibiting molecules, as well as the unexpected identification of a single functional Heparanase, suggest that the enzyme is a promising target for anti-cancer drug development.

DF can act both inhibiting the activity of the enzyme and down regulating its expression. The Heparanase activity and its possible inhibition can be determined by the heparan Degrading Enzyme Assay Kit whereas, its expression and possible down regulation can be valuated by Real-Time PCR.

### 3. Description of method

To evaluate the effect of DF either on Heparanase expression and its activity, the U266 and HMEC cells were incubated for 24h with DF at different concentrations or saline (control cells). After incubation with Defibrotide, the cells were washed with phosphate-buffered saline (PBS) pH7.4, and different U266 and HMEC samples were prepared for different experiments.

#### 3.1. Real Time PCR

##### 3.1.1. RNA isolation

RNA has been isolated from U266 and HMEC cells ($1.5 \times 10^5$ Cells/ml) treated with saline (control) or DF at doses of 150 and 400$\mu$g/ml for 24h. To isolate the RNA were used the RNeasy Mini Kit from Qiagen according the manufacture's instructions.

The 1% agarose gel electrophoresis, stained with Ethidium Bromide, was performed on all samples to check for presence of clear 28S and 18S ribosomal subunit bands.

##### 3.1.2. cDNA synthesis

Purified RNA, was used as a substrate for single-stranded cDNA synthesis using iScript™ cDNA Synthesis Kit (Bio-Rad) including: MuLV reverse transcriptase, random examers and dNTP mix. The incubation was carried out at 42°c' for 30 min. The template is the cDNA generated from reverse transcription reaction.

##### 3.1.3. Real-Time PCR

In order to perform the Real Time PCR was used the SYBER Green PCR Master Mix Reagent (SYBER Green PCR- Bio-Rad). Direct detection of polymerase chain reaction (PCR) product was monitored by measuring the increase in fluorescence caused by the binding of SYBER Green to double-stranded DNA.

Real Time PCR, using specific primers for Heparanase (forward 5'-TCACCATTGACGCCAACCT-3'; reverse 5'-CTTTGCAGAACCCAGGAGGAT-3'), was performed on the MyIQ PCR Sequence Detection System (Bio-Rad) designed for used with the SYBER Green PCR master mix reagents. The cycling parameters was 95°C for 3 min, 45 cycles at 95°C; 45°C; 72°C for 30s each and 72°C for 5 min. Data were acquired and processed with the MyIQ PCR software. The housekeeping actin transcript was used to normalized for the amount and quality of the RNAs.

#### 3.2. Heparanase Activity assay:

The Heparanase activity was measured in U266 extracts ($1 \times 10^5$ Cell/ml of extraction buffer) by a commercial Heparan Degrading Enzyme Kit (Takara-bio Inc.) according to manufacturer's instruction. The U266 cells have been treated with saline (control) or DF at doses of 50, 100 and 150$\mu$g/ml for 24h.

##### 3.2.1. Principle of method

Heparan Degrading Enzyme Assay Kit measure the activity of heparan degrading enzyme in cultured cells, utilizing the property that heparan-like molecules and bFGF (basic fibroblast growth factor) combine each other. CBD-FGF is a fusion protein of cell-binding domain of human fibronectin and human fibroblast growth factor (Takara-bio Inc.). This CBD-FGF is bound on a microtiterplate supplied in this kit, with captured by anti-fibronectin antibody having epitope in CBD region.

In addition, biotinylated heparan sulfate is used as a substrate of the enzyme. Since only undegraded substrate can combine to CBD-FGF, the detection of the remaining undegraded substrate by avidin-peroxidase realizes high sensitive measurement.

The reaction has been performed following the schematic steps bellow:

- Reaction of biotinylated heparan sulfate and sample
- Transfer of the reactant into well of CBD-FGF immobilized 96-well plate
- Reaction of remaining undegraded biotinylated heparan sulfate bound to CBD-FGF with avidin POD conjugate
- Color development by POD substrate

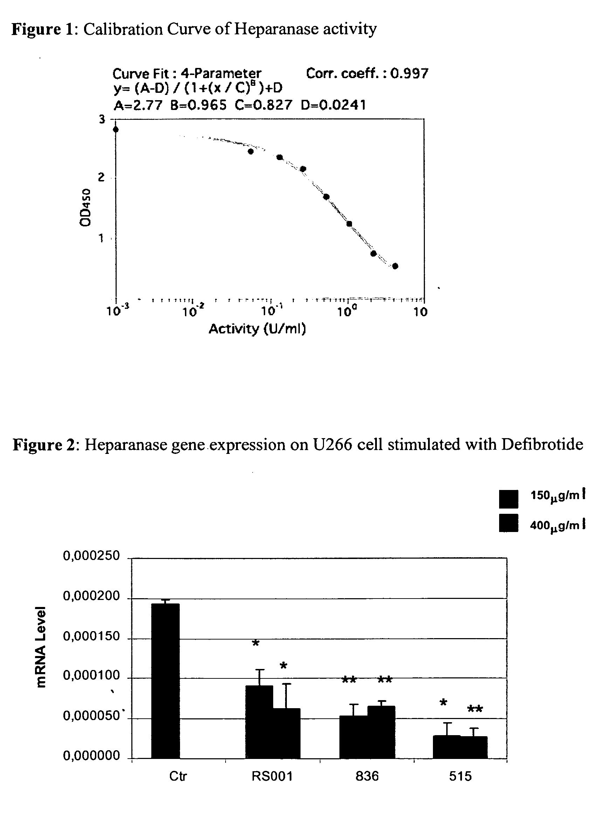

The calibration curve of Heparanase activity is reported in figure 1.

## 4. Results

### 4.1. Effect of Defibrotide on Heparanase gene expression

#### 4.1.1. Effect on myeloma tumor cells

Real-Time PCR was performed on cDNAs prepared from U266 cells treated with saline (control) or DF at dose of 150 and 400$\mu$g/ml. The experiments were performed in triplicate and the results are expressed as mRNA levels normalized by the housekeeping actin gene.

The results, which are summarized in figure 2, indicates that DF acts on U266 myeloma cells line through altering the Heparanase gene expression

#### 4.1.2. Effect on human microvascular endothelial cells

Real-Time PCR was performed on cDNAs prepared from HMEC cells treated with saline (control) or DF at dose of 150$\mu$g/ml. The experiments were performed in triplicate and the results are expressed as mRNA levels normalized by the housekeeping actin gene.

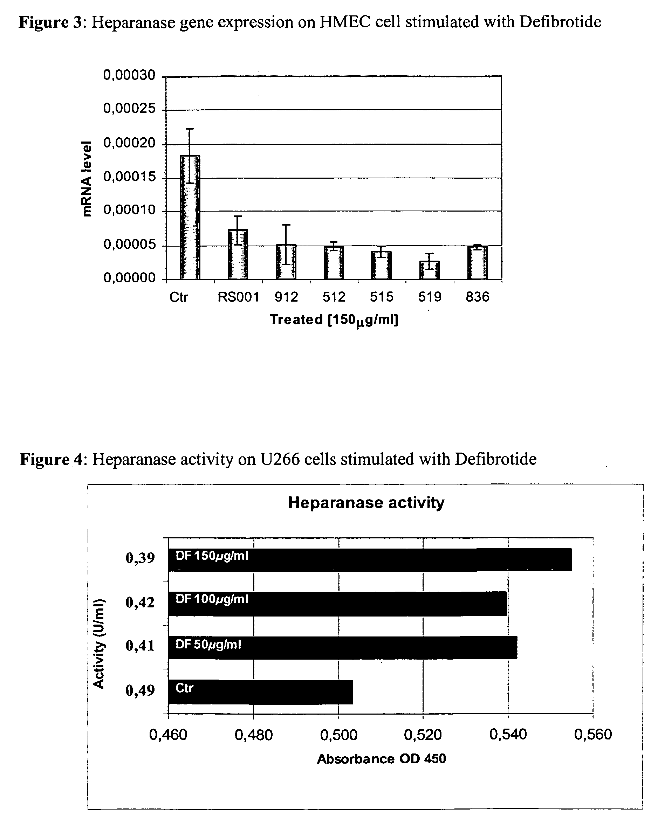

The results, which are summarized in figure 3, indicates that DF acts on Microvascular endothelial cells through altering the Heparanase gene expression

### 4.2. Effect of Defibrotide on enzymatic activity of Heparanase in myeloma cell line

The activity of Heparanase were measured using the Heparanse Degrading Enzyme Assay kit on U266 cells treated with saline (control) or Defibrotide at dose of 50, 100 and 150$\mu$g/ml. The experiments were performed in triplicate and the activity of Heparanase is shown with decrease of absorbance.

The results, which are summarized in figure 4, indicates that DF interferes on the Heparanase activity in the myeloma cell line.

## 5. Conclusions

Heparanase, an endoglyosidase involved in cleavage of heparan sulphate (HS), plays an important role in ECM degradation, facilitating the migration and extravasation of tumor cells and inflammatory leukocytes (15,16,17). It is believe that the inhibition of Heparanase may assist in the relief or cure of human illness including autoimmune and inflammatory disease such as arthritis and multiple sclerosis.

In our study, we have shown that Heparanase has a high expression and activity on myeloma cell line U266 and DF plays an important role either in down regulation of Heparanase gene and decrease of its enzymatic activity.

Important results were also obtained studying the human microvascular endothelial cells. Heparan sulphate (HS) is critical to the function of endothelial cells, which line blood vessels. For example, HS contribute to angiogenesis, tumor metastasis, and endothelial cell proliferation. In this context, Heparanase can alter the normal metabolism of endothelial cell heparan sulphate changing dramatically the function of endothelium. Our results showed on important role of DF in downregulation of Heparanase gene expression on HMEC cells.

In the light of these results, the object of the present invention is therefore represented by the use of DF for the manufacture of a medicament for the treatment of all those diseases which are or may be positively affected by the inhibition of Heparanase and/or by the downregulation of Heparanase gene expression, in particular on HMEC cells.

It is in fact widely believed that the inhibition of heparanase may assist in the relief or cure of human illnesses including autoimmune and/or inflammatory diseases such as arthritis and multiple sclerosis (18, 19, 20, 21, 22, 23).

The inhibition of heparanase will prevent the inflow of white blood cells that burrow between cells lining blood vessels resulting in painful inflammation. While inflammation is a normal immune response, the inhibition of heparanase to restrict the number of white blood cells invading a disease site may significantly relieve inflammation.

In particular, among inflammatory diseases, inhibition of heparanase will be particularly effective in treating kidney diseases. Heparan sulfate proteoglycans (HSPG) are in fact the 'glue' that helps to fill the spaces between proteins in tissues. In the kidney, these are particularly important because they influence the way that it acts as a filter of the blood. The kidneys are made up of a million sieves or filters named glomeruli. These sieves act to regulate the contents of the urine, and their integrity is essential to maintain health. The scaffold of these sieves is made up of many complex molecules including HSPG. HSPG act as "guards", ensuring excretion of unwanted substances into the urine but retention of proteins that are still required. Heparanase is believed to digest these "guards" (HSPG); consequently, substances normally kept within the circulation, are lost into the urine leading to proteinuria. If unchecked, this protein loss contributes to kidney disease progression and kidney failure. Different Works have confirmed that the active form of heparanase is markedly increased in disease. Heparanase blockade may prove to be beneficial in man, by preventing ongoing protein loss and arresting disease progression (24).

Finally, among autoimmune diseases, inhibition of heparanase will be particularly effective in treating diabete. Uncontrolled hyperglycemia is in fact the main risk factor in the development of diabetic vascular complications. The endothelial cells are the first cells targeted by hyperglycemia. The mechanism of endothelial injury by high glucose is still poorly understood. Heparanase production, induced by hyperglycemia, and subsequent degradation of heparan sulphate may contribute to endothelial injury. Han et al. suggested that high glucose may induce Heparanase upregulation which degrades HS causing cell injury and showed a link between hyperglycemia and Heparanase induction in diabetic complications (25).

As regards the methods of administering DF, they are not limiting for the purposes of the invention. That is to say, DF can be administered to mammals (and in particular to human beings) in accordance with the methods and the posologies known in the art; generally, it may be administered orally, intramuscularly, intraperitoneally, subcutaneously or intravenously, the last-mentioned route being the preferred one.

## 6. Bibliography

1. US-3,770,720
2. US-3,899,481
3. US-3,829,567
4. US-4,694,134
5. US-4,693,995
6. US-4,985,552
7. US-5,223,609
8. US-5,977,083
9. US-6,699,985
10. WO2005/023273
11. Richardson et al. Treatment of severe veno-occlusive disease with defibrotide:compassionate use results in response without significant toxicity in a high-risk population. Blood, 1998; 92: 737-44.
12. Richardson et al., Multi-institutional use of defibrotide in 88 patients after stem cell transplantation with severe veno-occlusive disease and multi-system organ failure: response without significant toxicity in a high risk population and factors predictive of outcome. Blood, 2002; 100(13):4337-4343.
13. Eissner et al., Fludarabine induces apoptosis, activation, and allogenicity in human endothelial and epithelial cells: protective effect of defibrotide. Blood, 2002; 100:334-340.
14. Falanga et al., Defibrotide reduces procoagulant activity and increases fibrinolytic properties of endothelial cells. Leukemia, 2003; 1636-42.
15. Parish et.al., Heparanase: a key enzyme involved in cell invasion. Biochem. Biophys. Acta., 2001; 1471(3):M99-MI08.
16. Vlodavsky et.al., Mammalian heparanase: gene cloning, expression and function in tumor progression and metastasis. Nature Medicine. 1999; (5):793-802.
17. Vlodavsky et al., Molecular properties and involvement of heparanase in cancer metastasis and angiogenesis.Clin. Invest. 2001; (108):341-347.
18. Hershkoviz et al., Differential effects of polysulfated polysaccharide on experimental encephalomyelitis, proliferation of autoimmune T cells, and inhibition of heparanase activity. J. Autoimmun. 1995; Oct;8(5): 741-750.
19 Irony-Tur-Sinai et al., A synthetic heparin-mimicking polyanionic compound inhibits central nervous system inflammation. J. Neurol Sci. 2003; Jan 15; 206(1): 49-57.
20. Parish et al., Treatment of central nervous system inflammation with inhibitors of basement membrane degradation. Immunol. Cell. Biol. 1998; Feb; 76(1):104-113.
21. Dempsey et al., Heparanase expression in in invasive trophoblasts and acute vascular damage, Glycobiology, vol. 10, n. 55, pp 467-475, 2000.
22. Brenchley, Antagonising angiogenesis in reumathoid artritis, Ann. Reum. Dis. Pp 71-74, 2001.
23. de Mestre et al., Regulation of inducible Heparanase gene trascription in activated T cells by early growth response 1, The Journal of Biological Chemistry, vol. 278, n. 50, pp 50377-50385, 2003.
24. Levidiotis et al., Heparanase inhibition reduces proteinuria in a model of accelerated anti-glomerular basement membrane antibody disease, Nephrology, Volume 10, Number 2, April 2005, pp. 167-173(7).
25. Han et al., Endothelial cell injury by high glucose and Heparanase is prevented by insulin, heparin and basic fibroblast growth factor, Cardiovascular Diabetology, August 2005.

**Claims**

1.  Use of defibrotide for the manufacture of a medicament for the treatment of diseaseses which are positively affected by the inhibition of Heparanse and/or by the downregulation of Heparanse gene expression, excluding cancer.

2.  Use according to claim 1, **characterized in that** said diseases are selected from autoimmune and/or inflammatory diseases.

3.  Use according to claim 2, **characterized in that** said diseases are selected from arthritis, multiple sclerosis, diabete and kidney diseases.

4.  Use according to claim 1, **characterized in that** said medicament is administered to a mammalian.

5.  Use according to claim 4, **characterized in that** said mammalian is a human being.

6.  Use according to claim 1, **characterized in that** Heparanase gene expression is inhibited in human microvascular endothelial cells.

7.  Use according to claim 1, **characterized in that** defibrotide is administered orally, intramuscularly, intraperitoneally, subcutaneously or intravenously.

8.  Use according to claim 1, **characterized in that** said medicament is in the form of an aqueous solution or suspension or in the form of a solid orally administrable formulation, such as a tablet.

9.  Use according to claim 8, **characterized in that** said medicament contains customary excipients and/or adjuvants.

10. Use according to claim 1, **characterized in that** defibrotide is of natural or synthetic origin.

**Figure 1**: Calibration Curve of Heparanase activity

Curve Fit : 4-Parameter     Corr. coeff. : 0.997
$y= (A-D) / (1+(x / C)^B )+D$
$A=2.77$  $B=0.965$  $C=0.827$  $D=0.0241$

**Figure 2**: Heparanase gene expression on U266 cell stimulated with Defibrotide

**Figure 3**: Heparanase gene expression on HMEC cell stimulated with Defibrotide

**Figure 4**: Heparanase activity on U266 cells stimulated with Defibrotide

**EP 1 872 787 A1**

European Patent
Office

**EUROPEAN SEARCH REPORT**

Application Number

EP 06 42 5436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 059 092 A1 (CRINOS INDUSTRIA FARMACO [IT] GENTIUM S P A [IT]) 13 December 2000 (2000-12-13) * paragraph [0033] * * paragraph [0006] * ----- | 1,2,4-10 | INV. A61K31/711 A61P13/12 A61P37/00 A61P3/08 |
| X | US 5 624 912 A (BURCOGLU ARSINUR [US] ET AL) 29 April 1997 (1997-04-29) * column 11, line 50 - column 12, line 21 * ----- | 1-10 | |
| X | US 4 649 134 A (BONOMINI VITTORIO [IT]) 10 March 1987 (1987-03-10) * claim 1 * ----- | 1-10 | |
| X | VINGOLO, ENZO MARIA ET AL: "Treatment of nonproliferative diabetic retinopathy with Defibrotide in noninsulin-dependent diabetes mellitus: A pilot study" ACTA OPHTHALMOLOGICA SCANDINAVICA , 77(3), 315-320 CODEN: AOSCFV; ISSN: 1395-3907, 1999, XP002411587 * the whole document * ----- | 1-10 | TECHNICAL FIELDS SEARCHED (IPC) A61K |

The present search report has been drawn up for all claims

| Place of search | Date of completion of the search | Examiner |
|---|---|---|
| Munich | 14 December 2006 | Giacobbe, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**                    EP 06 42 5436

This annex lists the patent family members relating to the patent documents cited in  the above-mentioned European search report.
The members are as contained in the European Patent Office EDP file on
The European Patent Office is in no way liable for these particulars which are merely given for the purpose of information.

14-12-2006

| Patent document cited in search report | | Publication date | Patent family member(s) | | Publication date |
|---|---|---|---|---|---|
| EP 1059092 | A1 | 13-12-2000 | AU | 3501099 A | 14-12-2000 |
| | | | CA | 2274419 A1 | 11-12-2000 |
| | | | JP | 2001002565 A | 09-01-2001 |
| | | | KR | 20010002244 A | 15-01-2001 |
| | | | US | 2002142029 A1 | 03-10-2002 |
| US 5624912 | A | 29-04-1997 | NONE | | |
| US 4649134 | A | 10-03-1987 | BE | 900563 A1 | 02-01-1985 |
| | | | CH | 661871 A5 | 31-08-1987 |
| | | | DE | 3433329 A1 | 28-03-1985 |
| | | | EP | 0137542 A2 | 17-04-1985 |
| | | | IT | 1170215 B | 03-06-1987 |
| | | | JP | 1922474 C | 07-04-1995 |
| | | | JP | 6047546 B | 22-06-1994 |
| | | | JP | 60084224 A | 13-05-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3770720 B
- US 3899481 B
- US 3829567 A
- US 4694134 B
- US 4693995 B
- US 4985552 B
- US 5223609 B
- US 5977083 B
- US 6699985 B
- WO 2005023273 A

### Non-patent literature cited in the description

- **RICHARDSON et al.** Treatment of severe veno-occlusive disease with defibrotide:compassionate use results in response without significant toxicity in a high-risk population. *Blood,* 1998, vol. 92, 737-44
- **RICHARDSON et al.** Multi-institutional use of defibrotide in 88 patients after stem cell transplantation with severe veno-occlusive disease and multi-system organ failure: response without significant toxicity in a high risk population and factors predictive of outcome. *Blood,* 2002, vol. 100 (13), 4337-4343
- **EISSNER et al.** Fludarabine induces apoptosis, activation, and allogenicity in human endothelial and epithelial cells: protective effect of defibrotide. *Blood,* 2002, vol. 100, 334-340
- **FALANGA et al.** Defibrotide reduces procoagulant activity and increases fibrinolytic properties of endothelial cells. *Leukemia,* 2003, 1636-42
- **PARISH.** Heparanase: a key enzyme involved in cell invasion. *Biochem. Biophys. Acta.,* 2001, vol. 1471 (3), M99-MI08
- **VLODAVSKY.** Mammalian heparanase: gene cloning, expression and function in tumor progression and metastasis. *Nature Medicine.,* 1999, 793-802
- **VLODAVSKY et al.** Molecular properties and involvement of heparanase in cancer metastasis and angiogenesis. *Clin. Invest.,* 2001, 341-347
- **HERSHKOVIZ et al.** Differential effects of polysulfated polysaccharide on experimental encephalomyelitis, proliferation of autoimmune T cells, and inhibition of heparanase activity. *J. Autoimmun.,* October 1995, vol. 8 (5), 741-750
- **IRONY-TUR-SINAI et al.** A synthetic heparin-mimicking polyanionic compound inhibits central nervous system inflammation. *J. Neurol Sci.,* 15 January 2003, vol. 206 (1), 49-57
- **PARISH et al.** Treatment of central nervous system inflammation with inhibitors of basement membrane degradation. *Immunol. Cell. Biol.,* February 1998, vol. 76 (1), 104-113
- **DEMPSEY et al.** Heparanase expression in in invasive trophoblasts and acute vascular damage. *Glycobiology,* 2000, vol. 10 (55), 467-475
- **BRENCHLEY.** Antagonising angiogenesis in reumathoid artritis. *Ann. Reum. Dis.,* 2001, 71-74
- **DE MESTRE et al.** Regulation of inducible Heparanase gene trascription in activated T cells by early growth response 1. *The Journal of Biological Chemistry,* 2003, vol. 278 (50), 50377-50385
- **LEVIDIOTIS et al.** Heparanase inhibition reduces proteinuria in a model of accelerated anti-glomerular basement membrane antibody disease. *Nephrology,* April 2005, vol. 10 (2), 167-173
- **HAN et al.** Endothelial cell injury by high glucose and Heparanase is prevented by insulin, heparin and basic fibroblast growth factor. *Cardiovascular Diabetology,* August 2005